# EUROPEAN PATENT SPECIFICATION

(11) **EP 3 821 798 B1**
(45) Date of publication and mention of the grant of the patent: **27.11.2024**
(21) Application number: 20205236.1
(22) Date of filing: 02.11.2020
(51) Int. Cl.: A61B 5/024, A61B 5/0245, A61B 5/08, A61B 5/0205, A61B 5/00

(54) **METHOD FOR GENERATING TRAINED MODEL, SYSTEM FOR GENERATING TRAINED MODEL, PROGRAM, AND ESTIMATION APPARATUS**
VERFAHREN ZUR ERZEUGUNG EINES TRAINIERTEN MODELLS, SYSTEM ZUR ERZEUGUNG EINES TRAINIERTEN MODELLS, PROGRAMM UND ABSCHÄTZVORRICHTUNG
PROCÉDÉ DE GÉNÉRATION DE MODÈLE FORMÉ, SYSTÈME DE GÉNÉRATION DE MODÈLE FORMÉ, PROGRAMME ET APPAREIL D'ESTIMATION

(30) Priority: 14.11.2019 JP 2019206134
(43) Date of publication of application: 19.05.2021
(73) Proprietor: Nihon Kohden Corporation, Shinjuku-ku Tokyo (JP)
(72) Inventor: KAWASHIMA, Takuya, Tokorozawa-shi, Saitama (JP); MATSUZAWA, Wataru, Tokorozawa-shi, Saitama (JP); SANO, Hiroto, Tokorozawa-shi, Saitama (JP)
(74) Representative: Grünecker Patent- und Rechtsanwälte PartG mbB

(56) References cited:
- US-A1- 2012 302 900
- US-A1- 2015 327 781
- DE MORAIS BORGES GABRIEL ET AL: "Bayesian fusion of multiple sensors for reliable heart rate detection", 2014 IEEE INTERNATIONAL INSTRUMENTATION AND MEASUREMENT TECHNOLOGY CONFERENCE (I2MTC) PROCEEDINGS, IEEE, 12 May 2014 (2014-05-12), pages 1310 - 1313, XP032620894, DOI: 10.1109/I2MTC.2014.6860957

## Description

### TECHNICAL FIELD

The presently disclosed subject matter relates to a method for generating a trained model applied to an estimation apparatus configured to estimate a probability that a value of a predetermined physiological parameter is correctly calculated based on waveform data acquired from a subject being tested, and a system configured to generate the trained model. The presently disclosed subject matter also relates to the estimation apparatus, and a computer program that is executed in the estimation apparatus.

### BACKGROUND ART

JP-A-2018-102671 discloses a patient monitor. A value of a physiological parameter of a patient or a subject being tested acquired from a sensor and the like attached to the subject being tested is provided for monitoring and displaying by the patient monitor. When the value indicates deviation from a normal state, the patient monitor outputs an alarm for notification to a healthcare professional.
De Morais Borges Gabriel et al.: "Bayesian fusion of multiple sensors for reliable heart rate detection", 2014 IEEE INTERNATIONAL INSTRUMENTATION AND MEASUREMENT TECHNOLOGY CONFERENCE (I2MTC) PROCEEDINGS, IEEE, 12 May 2014, pages 1310-1313, discloses a bayesian approach to fuse information from multiple sensors for reliable heart rate detection.
US 2012/0302900 deals with an efficient analysis of a cyclic physiological signal which is especially suitable for continuous monitoring of patients where a trend of a reliable physiological signal is more important than an instantaneous measurement of a reliable physiological signal on a peak-by-peak basis.

An object of the presently disclosed subject matter is to accurately extract a value of a physiological parameter resulting from a physiological phenomenon of a subject being tested.

### SUMMARY

In a first aspect for achieving the above-described object, a method for generating a trained model is applied to an estimation apparatus for estimating a probability that a value of a predetermined physiological parameter is correctly calculated based on waveform data acquired from a subject being tested. The method includes: acquiring first data corresponding to a value of a first physiological parameter that has been correctly calculated from first waveform data; inputting second waveform data to an algorithm automatically calculating a value of a second physiological parameter acquired from input waveform data and to output second data; generating third data including a training label indicating whether the value of the second physiological parameter corresponding to the second data is a correct answer or an incorrect answer by comparing the second data with the first data; and training a neural network by using the second waveform data and the third data, to generate a trained mode which, when applied to said estimation apparatus, configures the apparatus to estimate said probability.

In a second aspect for achieving the above-described object, a system for generating a trained model to be applied to an estimation apparatus for estimating a probability that a value of a predetermined physiological parameter is correctly calculated based on waveform data acquired from a subject being tested. The system includes: a training data generation apparatus; and a trained model generation apparatus. The training data generation apparatus includes: a first input interface configured to receive first data corresponding to a value of a first physiological parameter that has been correctly calculated from first waveform data, and second data calculated by inputting second waveform data to an algorithm automatically calculating a value of a second physiological parameter acquired from input waveform data and to output second data; first one or more processors configured to generate third data including a training label indicating whether the value of the second physiological parameter corresponding to the second data is a correct answer or an incorrect answer by comparing the second data with the first data; and an output interface configured to output the third data. The trained model generation apparatus includes: a second input interface configured to receive the second waveform data and the third data; and second one or more processors configured to train a neural network by using the second waveform data and the third data to generate a trained mode which, when applied to said estimation apparatus, configures said apparatus to estimate said probability

In a third aspect for achieving the above-described object, a computer program is executed in a system including a training data generation apparatus and a trained model generation apparatus. When executed, the computer program causes the training data generation apparatus to: receive first data corresponding to a value of a first physiological parameter that has been correctly calculated from first waveform data, and second data acquired by inputting second waveform data to an algorithm automatically calculating a value of a second physiological parameter acquired from input waveform data and output second data; generate third data including a training label indicating whether the value of the second physiological parameter corresponding to the second data is a correct answer or an incorrect answer by comparing the second data with the first data; and output the third data. The computer program causes the trained model generation apparatus to: receive the second waveform data and the third data; and train a neural network by using the second waveform data and the third data to generate a trained model applied to an estimation apparatus configured to estimate a probability that a value of a predetermined physiological parameter is correctly calculated based on waveform data acquired from a subject being tested.

In a fourth aspect for achieving the above-described object, an estimation apparatus includes: an input interface configured to receive waveform data acquired from a subject being tested; one or more processors configured to generate estimation data corresponding to a probability that a value of a predetermined physiological parameter is correctly calculated based on the waveform data; and an output interface configured to output the estimation data. The one or more processors are configured to generate the estimation data by using the trained model generated by the method described above.

In a fifth aspect for achieving the above-described object, a computer program is executed by an estimation apparatus configured to estimate a probability that a value of a predetermined physiological parameter is correctly calculated based on waveform data acquired from a subject being tested. When executed, the computer program causes the estimation apparatus to: receive waveform data acquired from the subject being tested; input the waveform data to the trained model generated by the method described above; generate estimation data corresponding to the probability, based on an output from the trained model; and output the estimation data.

### BRIEF DESCRIPTION OF DRAWINGS

FIG. 1 exemplifies generation and use of a trained model in accordance with a first embodiment;
FIG. 2 exemplifies a relation between electrocardiogram waveform data and first heart rate data;
FIG. 3 exemplifies a relation between the electrocardiogram waveform data and second heart rate data;
FIG. 4 exemplifies data illustrating change over time in heart rate acquired from a subject being tested;
FIG. 5 exemplifies data illustrating change over time in heart rate after being processed by an estimation apparatus;
FIG. 6 exemplifies generation and use of a trained model in accordance with a second embodiment; and
FIG. 7 exemplifies generation and use of a trained model in accordance with a third embodiment.

### DESCRIPTION OF EMBODIMENTS

Hereinafter, examples of embodiments will be described in detail with reference to the accompanying drawings.

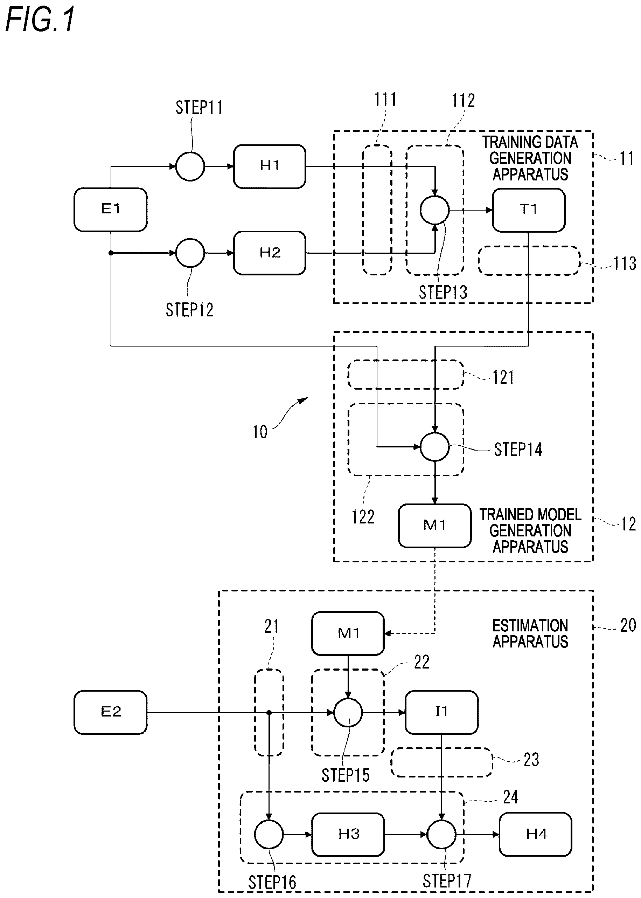

FIG. 1 exemplifies a functional configuration of a trained model generation system 10 in accordance with a first embodiment.

The trained model generation system 10 is a system configured to generate a trained model M1 that is a processing algorithm, which is executed in an estimation apparatus 20 to be described later. The trained model generation system 10 can include a training data generation apparatus 11 and a trained model generation apparatus 12.

The training data generation apparatus 11 can include an input interface 111. The input interface 111 is configured to receive first heart rate data H1 and second heart rate data H2. The input interface 111 is an example of the first input interface.

The first heart rate data H1 is data indicative of a value of a heart rate correctly calculated from electrocardiogram waveform data E1. The electrocardiogram waveform data E1 is an example of the first waveform data. The heart rate is an example of the first physiological parameter. The first heart rate data H1 is an example of the first data.

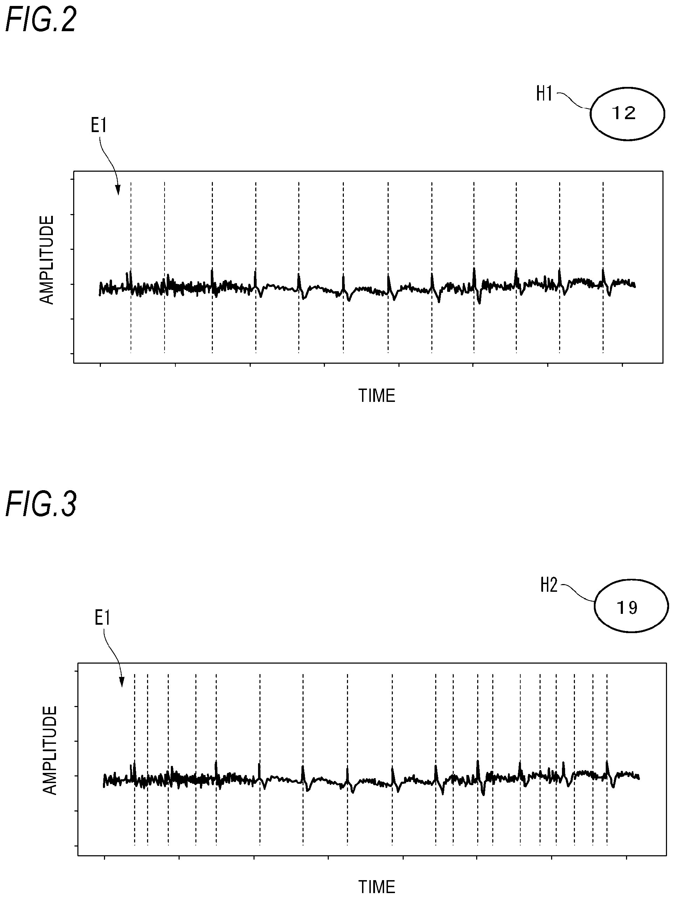

FIG. 2 exemplifies a relation between the electrocardiogram waveform data E1 and the first heart rate data H1.

The electrocardiogram waveform data E1 indicates change over time in physiological electrical activity of a heart of a subject being tested. The electrocardiogram waveform data E1 changes depending on pulsation of the heart. By counting the number of times of the change per minute, the first heart rate data H1 is calculated. In FIG. 2, the broken lines extending vertically indicate portions of the electrocardiogram waveform data E1 used for counting the heart rate. In FIG. 2, the heart rate is counted 12 times within a time (for example, for 10 seconds) in which the waveform is displayed.

That is, when generating the trained model M1, processing (STEP 11, in FIG. 1) of acquiring the first heart rate data H 1 from the electrocardiogram waveform data E1 is performed. The counting of the heart rate corresponding to the first heart rate data H1 may be performed by visually checking the electrocardiogram waveform data E1 or may be performed using appropriate counting software. As the electrocardiogram waveform data E1, waveform data that is actually acquired from the subject being tested by using an electrocardiogram sensor or the like may be used or waveform data that is published as a database for performance evaluation of an electrocardiogram monitor may be used.

The second heart rate data H2 is data indicative of a value of the heart rate automatically calculated from the electrocardiogram waveform data E1 by a specific heart rate calculation algorithm. The heart rate calculation algorithm may be executed by one or more processors mounted in an electrocardiogram monitor, for example. The heart rate calculation algorithm is configured to automatically calculate a heart rate, based on a feature of a waveform shape of input electrocardiogram waveform data, and the like. The electrocardiogram waveform data E1 is an example of the second waveform data. The heart rate is an example of the second physiological parameter. The second heart rate data H2 is an example of the second data.

That is, when generating the trained model M1, processing (STEP 12, in FIG. 1) of acquiring the second heart rate data H2 from the electrocardiogram waveform data E1 is performed.

FIG. 3 exemplifies a relation between the electrocardiogram waveform data E1 and the second heart rate data H2.

The electrocardiogram waveform data E1 exemplified in FIG. 3 is the same as the electrocardiogram waveform data E1 exemplified in FIG. 2. In the example based on the same electrocardiogram waveform data E1 and illustrated in FIG. 3, the heart rate is counted 19 times, not 12 times. This means that the heart rate calculation algorithm used to acquire the second heart rate data H2 may perform the counting based on an erroneously detected heart rate.

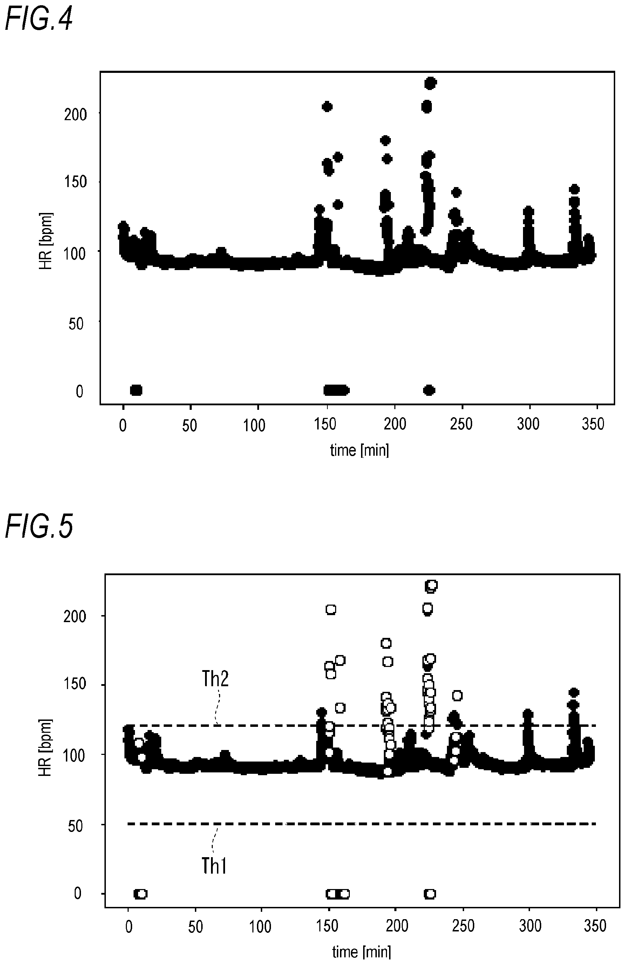

FIG. 4 exemplifies change over time in heart rate automatically calculated by inputting, to the heart rate calculation algorithm, the electrocardiogram waveform data E1 actually acquired from the subject being tested. A plurality of points included in the graph each corresponds to heart rate detected within a predetermined time (for example, one minute).

In FIG. 5, erroneously calculated values of the values of the plurality of heart rates exemplified in FIG. 4 are indicated by white circles. One method capable of excluding the erroneously calculated heart rate data is to set a threshold value range for the heart rate. For example, heart rate data of which heart rate is not included between a lower limit threshold value Th1 and an upper limit threshold value Th2 may be excluded. However, as illustrated in FIG. 5, the erroneously calculated heart rate data may exist even within the threshold value range, and the correctly calculated heart rate data may exist beyond the threshold value range. The former is based on misanalysis by the heart rate calculation algorithm, and the latter corresponds to a case where the heart rate rises due to any physiological phenomenon of the subject being tested. The misanalysis of the heart rate calculation algorithm may be caused due to a phenomenon (so-called, double count) that a T wave having a high amplitude is erroneously recognized as a QRS wave and the heart rate is more counted, for example. The misanalysis of the heart rate calculation algorithm may also be caused due to body motion of the subject being tested and external noises such as deterioration and detaching of an electrode attached to the subject being tested, and the like.

As exemplified in FIG. 1, the training data generation apparatus 11 can include one or more processors 112. The one or more processors 112 are configured to compare the second heart rate data H2 with the first heart rate data H1, thereby generating training data T1 including a training label indicating whether a value of the heart rate corresponding to the second heart rate data H2 is a correct answer or an incorrect answer. The one or more processors 112 are an example of the first one or more processors. The training data T1 is an example of the third data.

When the first heart rate data H1 exemplified in FIG. 2 and the second heart rate data H2 exemplified in FIG. 3 are provided for comparison, the training data T1 includes a training label indicating that the heart rate corresponding to the second heart rate data H2 is an incorrect answer.

In order to include a training label indicative of a correct answer, it is not necessarily required that the heart rate corresponding to the first heart rate data H1 should coincide with the heart rate corresponding to the second heart rate data H2. When an error of the heart rate corresponding to the second heart rate data H2 with respect to the heart rate corresponding to the first heart rate data H1 is within an allowable range, the training data T1 can include training data indicative of a correct answer.

That is, when generating the trained model M1, processing (STEP 13, in FIG. 1) of comparing the second heart rate data H2 corresponding to the heart rate automatically calculated by the heart rate calculation algorithm with the first heart rate data H1 to generate the training data T1 including the training label indicating whether the heart rate is a correct answer or an incorrect answer is performed.

The one or more processors 112 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 112 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The one or more processors 112 may also be implemented by a combination of one or more general-purpose microprocessor and a dedicated integrated circuit.

As illustrated in FIG. 1, the training data generation apparatus 11 can include an output interface 113. The output interface 113 is configured to output the training data T1 generated by the one or more processors 112.

The trained model generation apparatus 12 can include an input interface 121. The input interface 121 is configured to receive the electrocardiogram waveform data E1 and the training data T1. The input interface 121 is an example of the second input interface.

The trained model generation apparatus 12 can include one or more processors 122. The one or more processors 122 are configured to train a neural network by using the electrocardiogram waveform data E1 and the training data T1, thereby generating the trained model M1. The one or more processors 122 are an example of the second one or more processors.

The trained model M1 is generated as a processing algorithm that uses electrocardiogram waveform data as an input and outputs estimation data corresponding to a probability that the heart rate will be correctly calculated based on the electrocardiogram waveform data. The estimation data may be associated with a score (for example, any one of values 1 to 5) corresponding to a calculated probability, for example.

That is, when generating the trained model M1, processing (STEP 14, in FIG. 1) of training a neural network by using the electrocardiogram waveform data E1 and the training data T1 is performed. As processing learned by the neural network, a method relating to a well-known supervised learning is used as appropriate.

The one or more processors 122 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 122 may also be implemented by a dedicated integrated circuit such as a microcontroller, an ASIC, an FPGA, a TPU and the like capable of executing the computer program. In this case, the computer program is pre-installed in a storage device included in the dedicated integrated circuit. The storage device is an example of the storage medium in which the computer program is stored. The one or more processors 122 may also be implemented by a combination of one or more general-purpose microprocessor and a dedicated integrated circuit.

When the training data generation apparatus 11 and the trained model generation apparatus 12 are provided as apparatuses independent of each other, the output interface 113 of the training data generation apparatus 11 and the input interface 121 of the trained model generation apparatus 12 can be connected so as to enable wired communication or wireless communication. That is, the output interface 113 and the input interface 121 may be physical communication interfaces.

The training data generation apparatus 11 and the trained model generation apparatus 12 may also be functional entities implemented in the same apparatus. In this case, at least some of the functions of the one or more processors 112 of the training data generation apparatus 11 can be implemented by the one or more processors 122 of the trained model generation apparatus 12. Also, the output interface 113 and the input interface 121 may be logical interfaces.

According to the configuration as described above, the heart rate automatically calculated by the heart rate calculation algorithm is compared with the heart rate known as being correctly calculated. Therefore, the training label obtained as a result of the comparison can reflect a tendency or habit of the heart rate calculation algorithm correctly or erroneously calculating the heart rate with respect to the input electrocardiogram waveform data E1. Since the neural network is trained using the training data T1 including the training label, the generated trained model M1 can accurately estimate a probability that the heart rate will be correctly calculated when any electrocardiogram waveform data is input to the heart rate calculation algorithm. In other words, estimation accuracy as to discrimination between the value of the heart rate resulting from the physiological phenomenon of the subject being tested and the value of the heart rate resulting from the misanalysis of the heart rate calculation algorithm increases.

In the present example, the electrocardiogram waveform data E1 that is used so as to acquire the first heart rate data H1 corresponding to the value of the correctly calculated heart rate also serves as the electrocardiogram waveform data that is input to the heart rate calculation algorithm so as to acquire the second heart rate data H2 provided for comparison with the first heart rate data H1. Since the physiological parameters provided for comparison so as to generate the training data T1 are all the heart rate, the determination as to a correct answer or an incorrect answer is performed based on the same electrocardiogram waveform data E1, so that the learning accuracy can be improved.

As exemplified in FIG. 1, the trained model M1 generated by the trained model generation apparatus 12 is applied to the estimation apparatus 20.

The estimation apparatus 20 can include an input interface 21. The input interface 21 is configured to receive electrocardiogram waveform data E2 acquired from the subject being tested via an electrocardiogram sensor and the like.

The estimation apparatus 20 can include one or more processors 22. The one or more processors 22 are configured to estimate a probability that the heart rate will be correctly calculated from the electrocardiogram waveform data E2. The trained model M1 is a processing algorithm that is executed for the above-described estimation by the one or more processors 22. In this case, the trained model M1 uses the electrocardiogram waveform data E2 as an input, and outputs estimation data I1 corresponding to a probability that the heart rate will be correctly calculated based on the electrocardiogram waveform data E2.

That is, the estimation apparatus 20 is configured to execute processing (STEP 15, in FIG. 1) of estimating a probability that the heart rate will be correctly calculated based on the electrocardiogram waveform data E2 acquired from the subject being tested.

The one or more processors 22 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 22 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The one or more processors 22 may also be implemented by a combination of one or more general-purpose microprocessor and a dedicated integrated circuit.

The estimation apparatus 20 can include an output interface 23. The output interface 23 is configured to output the estimation data I1.

According to the configuration as described above, since the trained model M1, which can accurately estimate a probability that the heart rate will be correctly calculated when the electrocardiogram waveform data is input to the heart rate calculation algorithm, is used, estimation as to discrimination between the value of the heart rate resulting from the physiological phenomenon of the subject being tested and the value of the heart rate resulting from the misanalysis of the heart rate calculation algorithm can be accurately performed by the estimation apparatus 20.

The estimation apparatus 20 can include a data processing device 24. The data processing device 24 is configured to execute the heart rate calculation algorithm for automatically calculating the heart rate, based on the electrocardiogram waveform data E2 received by the input interface 21. In the present example, the heart rate calculation algorithm that is executed by the data processing device 24 is the same as the heart rate calculation algorithm used so as to acquire the second heart rate data H2 when generating the trained model M1. Thereby, heart rate data H3 corresponding to the heart rate automatically calculated based on the electrocardiogram waveform data E2 is generated.

That is, the data processing device 24 is configured to execute processing (STEP 16, in FIG. 1) of generating the heart rate data H3 corresponding to the heart rate automatically calculated based on the electrocardiogram waveform data E2 acquired from the subject being tested. The heart rate data H3 can be presented in an aspect as exemplified in FIG. 4.

In addition, the data processing device 24 is configured to generate processed heart rate data H4 by applying the processing based on the estimation data I1 output from the output interface 23 to the heart rate data H3.

That is, the data processing device 24 is configured to execute processing (STEP 17, in FIG. 1) based on a probability that the heart rate will be correctly calculated based on the electrocardiogram waveform data E2, for the heart rate automatically calculated based on the electrocardiogram waveform data E2 acquired from the subject being tested.

For example, the processed heart rate data H4 may be configured so that data of the heart rate data H3 of which the probability that the heart rate is correctly calculated exceeds a predetermined threshold value, and data of which the probability does not exceed the predetermined threshold value are displayed with colors different from each other. The display based on the processed heart rate data H4 configured in this way may be presented in an aspect as exemplified in FIG. 5. In FIG. 5, the data of which the probability that the heart rate is correctly calculated exceeds the predetermined threshold value is indicated by black circles, and the data of which the probability that the heart rate is correctly calculated does not exceed the predetermined threshold value is indicated by white circles. When the aspects are different depending on whether the probability exceeds a predetermined threshold value, shapes of symbols or presence or absence of blinking indicative of the data may be appropriately selected.

Alternatively, the processed heart rate data H4 may be configured so that only data of the heart rate data H3 of which the probability that the heart rate is correctly calculated exceeds a predetermined threshold value is displayed. In this case, only the data represented with the black circles in FIG. 5 is presented for display. In contrast, the processed heart rate data H4 may also be configured so that only data of the heart rate data H3 of which the probability that the heart rate is correctly calculated does not exceed the predetermined threshold value is displayed. In this case, only the data represented with the white circles in FIG. 5 is presented for display.

The estimation apparatus 20 can include a display device (not illustrated). In this case, the display corresponding to the processed heart rate data H4 is presented to the display device. The display corresponding to the processed heart rate data H4 may be performed in an external apparatus including a display device. In this case, the estimation apparatus 20 is configured to transmit the processed heart rate data H4 to the external apparatus via a communication interface (not illustrated).

The data processing device 24 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The data processing device 24 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The data processing device 24 may also be implemented by a combination of one or more general-purpose microprocessor and a dedicated integrated circuit.

When the one or more processors 22 and the data processing device 24 are provided as devices independent of each other, the output interface 23 may be a physical communication interface configured to relay data communication therebetween. The one or more processors 22 and the data processing device 24 may also be functional entities implemented in the same control device. In this case, the output interface 23 may be a logical interface.

According to the configuration as described above, it is possible to accurately extract a value of the heart rate resulting from the physiological phenomenon of the subject being tested by using a highly accurate estimation result about the probability that the heart rate will be correctly calculated when the electrocardiogram waveform data E2 acquired from the subject being tested is input to the heart rate calculation algorithm.

In the present example, the estimation apparatus 20 is configured to estimate the probability that the heart rate will be correctly calculated based on the electrocardiogram waveform data E2 acquired from the subject being tested. The trained model M1 that is used for estimation is generated using the first heart rate data H1 and the second heart rate data H2 acquired from the common electrocardiogram waveform data E1.

As another example, the estimation apparatus 20 may be configured to estimate a probability that a respiration rate will be correctly calculated based on capnogram data acquired from the subject being tested. The capnogram data is acquired from the subject being tested by using a capnometer and the like. The capnogram data corresponds to change over time in carbon dioxide concentration in exhaled breath of the subject being tested. The respiration rate is a number of respirations made for a predetermined time by the subject being tested. The capnogram data is an example of the waveform data. The respiration rate is an example of the physiological parameter.

In this case, the trained model M1 that is used for estimation by the estimation apparatus 20 is generated based on comparison of first respiration rate data and second respiration rate data acquired from the common capnogram data. The first respiration rate data is data indicative of a respiration rate correctly calculated from the capnogram data. The second respiration rate data is data indicative of a respiration rate automatically calculated from the same capnogram data by a specific respiration rate calculation algorithm. The first respiration rate data is an example of the first data. The second respiration rate data is an example of the second data. The respiration rate calculation algorithm that is used in the estimation apparatus 20 is the same as the respiration rate calculation algorithm that is used so as to acquire the second respiration rate data.

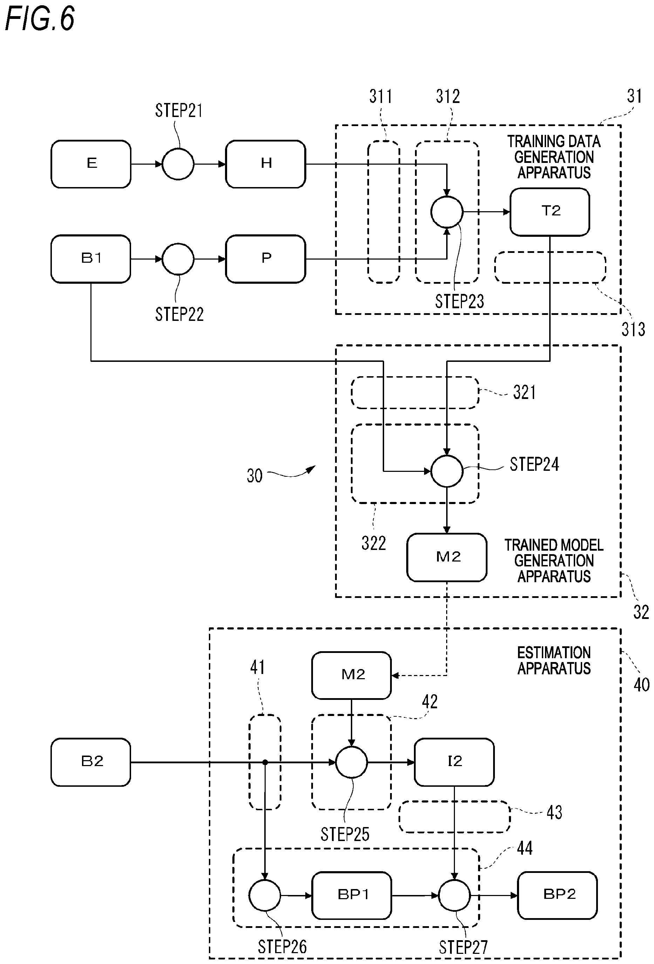

FIG. 6 exemplifies a functional configuration of a trained model generation system 30 in accordance with a second embodiment. The trained model generation system 30 is a system configured to generate a trained model M2 that is a processing algorithm, which is executed in an estimation apparatus 40 to be described later. The trained model generation system 30 can include a training data generation apparatus 31 and a trained model generation apparatus 32.

The training data generation apparatus 31 can include an input interface 311. The input interface 311 is configured to receive heart rate data H and pulse rate data P. The input interface 311 is an example of the first input interface.

The electrocardiogram waveform data E is acquired from the subject being tested by using an electrocardiogram sensor and the like. The electrocardiogram waveform data E corresponds to a physiological electrical activity of the heart of the subject being tested. The heart rate data H is data indicative of heart rates correctly calculated from the electrocardiogram waveform data E. The electrocardiogram waveform data E is an example of the first waveform data. The heart rate is an example of the first physiological parameter. The heart rate data H is an example of the first data.

That is, when generating the trained model M2, processing (STEP 21, in FIG. 6) of acquiring the heart rate data H from the electrocardiogram waveform data E is performed. The counting of the heart rate corresponding to the heart rate data H may be performed by visually checking the electrocardiogram waveform data E or may be performed using appropriate counting software.

Invasive arterial pressure waveform data B1 is acquired from the subject being tested by using a catheter and the like. The electrocardiogram waveform data E and the invasive arterial pressure waveform data B 1 are required to be acquired from the same subject being tested at the same time. The invasive arterial pressure waveform data B1 corresponds to change over time in invasive arterial pressure value of the subject being tested.

The pulse rate data P is data indicative of a value of a pulse rate automatically calculated from the invasive arterial pressure waveform data B1 by a specific pulse rate calculation algorithm. The pulse rate calculation algorithm may be executed by one or more processors mounted in the patient monitor, for example. The pulse rate calculation algorithm is configured to automatically calculate a pulse rate, based on variation timing of a blood pressure value of the input invasive arterial pressure waveform data, and the like, for example. The invasive arterial pressure waveform data B1 is an example of the second waveform data. The pulse rate is an example of the second physiological parameter. The pulse rate data P is an example of the second data.

That is, when generating the trained model M2, processing (STEP 22, in FIG. 6) of acquiring the pulse rate data P from the invasive arterial pressure waveform data B1 is performed.

As exemplified in FIG. 6, the training data generation apparatus 31 can include one or more processors 312. The one or more processors 312 are configured to compare the pulse rate data P with the heart rate data H, thereby generating training data T2 including a training label indicating whether a value of a pulse rate corresponding to the pulse rate data P is a correct answer or an incorrect answer. The one or more processors 312 is an example of the first one or more processors. The training data T2 is an example of the third data.

In order to include a training label indicative of a correct answer, it is not necessarily required that the heart rate corresponding to the heart rate data H should coincide with the pulse rate corresponding to the pulse rate data P. When an error of the pulse rate corresponding to the pulse rate data P with respect to the heart rate corresponding to the heart rate data H is within an allowable range, the training data T2 can include training data indicative of a correct answer.

That is, when generating the trained model M2, processing (STEP 23, in FIG. 6) of comparing the pulse rate data P corresponding to the pulse rate automatically calculated by the pulse rate calculation algorithm with the heart rate data H to generate the training data T2 including the training label indicating whether the pulse rate is a correct answer or an incorrect answer is performed.

The one or more processors 312 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 312 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The one or more processors 312 may also be implemented by a combination of one or more general-purpose microprocessor and a dedicated integrated circuit.

As exemplified in FIG. 6, the training data generation apparatus 31 can include an output interface 313. The output interface 313 is configured to output the training data T2 generated by the one or more processors 312.

The trained model generation apparatus 32 can include an input interface 321. The input interface 321 is configured to receive the invasive arterial pressure waveform data B1 and the training data T2. The input interface 321 is an example of the second input interface.

The trained model generation apparatus 32 can include one or more processors 322. The one or more processors 322 are configured to generate the trained model M2 by training the neural network with the invasive arterial pressure waveform data B1 and the training data T2. The one or more processors 322 are an example of the second one or more processors.

The trained model M2 is generated as a processing algorithm that uses invasive arterial pressure waveform data as an input and outputs estimation data corresponding to a probability that the pulse rate will be correctly calculated based on the invasive arterial pressure waveform data. The estimation data may be associated with a score (for example, any one of values 1 to 5) corresponding to a calculated probability, for example.

That is, when generating the trained model M2, processing (STEP 24, in FIG. 6) of training the neural network by using the invasive arterial pressure waveform data B1 and the training data T2 is performed. As processing learned by the neural network, a method relating to a well-known supervised learning is used as appropriate.

The one or more processors 322 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 322 may also be implemented by a dedicated integrated circuit such as a microcontroller, an ASIC, an FPGA, a TPU and the like capable of executing the computer program. In this case, the computer program is pre-installed in a storage device included in the dedicated integrated circuit. The storage device is an example of the storage medium in which the computer program is stored. The one or more processors 322 may also be implemented by a combination of one or more general-purpose microprocessor and a dedicated integrated circuit.

When the training data generation apparatus 31 and the trained model generation apparatus 32 are provided as apparatuses independent of each other, the output interface 313 of the training data generation apparatus 31 and the input interface 321 of the trained model generation apparatus 32 can be connected so as to enable wired communication or wireless communication. That is, the output interface 313 and the input interface 321 may be physical communication interfaces.

The training data generation apparatus 31 and the trained model generation apparatus 32 may also be functional entities implemented in the same apparatus. In this case, at least some of the functions of the one or more processors 312 of the training data generation apparatus 31 can be implemented by the one or more processors 322 of the trained model generation apparatus 32. Also, the output interface 313 and the input interface 321 may be logical interfaces.

In general, since the invasive arterial pressure is measured during surgery or for severely ill patients, the measurement is less affected by body motion of the subject being tested. However, the pulse rate and the invasive arterial pressure value may not be correctly calculated due to atmospheric pressure open zero-point calibration and the like. In the meantime, the effects of these events on the electrocardiogram waveform are relatively small. According to the configuration as described above, the pulse rate automatically calculated by the pulse rate calculation algorithm is compared with the heart rate known as being correctly calculated. Therefore, the training label obtained as a result of the comparison can reflect a tendency or habit of the pulse rate calculation algorithm correctly or erroneously calculating the pulse rate with respect to the input invasive arterial pressure waveform data B 1. Since the neural network is trained using the training data T2 including the training label, the generated trained model M2 can accurately estimate a probability that the pulse rate will be correctly calculated when any invasive arterial pressure waveform data is input to the pulse rate calculation algorithm. In other words, estimation accuracy as to discrimination between the value of the pulse rate resulting from the physiological phenomenon of the subject being tested and the value of the pulse rate resulting from the misanalysis of the pulse rate calculation algorithm increases.

In the present example, in order to calculate the heart rate and the pulse rate that are used for generation of the trained model M2, the electrocardiogram waveform data E and the invasive arterial pressure waveform data B1 are acquired from the same subject being tested. That is, in order to generate the trained model M2, two physiological parameters of which types are different are used, and the two physiological parameters are acquired by two different methods. The pulse rate may be calculated based on the pulse wave waveform data. The pulse wave waveform data may be acquired by noninvasively measuring change over time in absorbency of blood with a pulse photometry probe and the like. Alternatively, the pulse wave waveform data may be acquired by measuring change over time in internal pressure of a cuff with a noninvasive arterial pressure meter and the like.

As exemplified in FIG. 6, the trained model M2 generated by the trained model generation apparatus 32 is applied to the estimation apparatus 40.

The estimation apparatus 40 can include an input interface 41. The input interface 41 is configured to receive the invasive arterial pressure waveform data B2 acquired from the subject being tested via a catheter, and the like.

The estimation apparatus 40 can include one or more processors 42. The one or more processors 42 are configured to estimate a probability that an invasive arterial pressure value will be correctly calculated from the invasive arterial pressure waveform data B2. The trained model M2 is a processing algorithm that is executed for the above-described estimation by the one or more processors 42. In this case, the trained model M2 uses the invasive arterial pressure waveform data B2, as an input, and outputs estimation data I2 corresponding to a probability that an invasive arterial pressure value will be correctly calculated based on the invasive arterial pressure waveform data B2.

That is, the estimation apparatus 40 is configured to execute processing (STEP 25, in FIG. 6) of estimating a probability that an invasive arterial pressure value will be correctly calculated based on the invasive arterial pressure waveform data B2 acquired from the subject being tested.

The one or more processors 42 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 42 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The one or more processors 42 may also be implemented by a combination of one or more general-purpose microprocessor and a dedicated integrated circuit.

The estimation apparatus 40 can include an output interface 43. The output interface 43 is configured to output the estimation data 12.

The trained model M2 generated by the trained model generation system 30 is configured to estimate a probability that a pulse rate will be correctly calculated based on the input invasive arterial pressure waveform data. In the meantime, the probability that the invasive arterial pressure value will be correctly calculated based on the invasive arterial pressure waveform data acquired from the subject being tested is estimated by the estimation apparatus 40, and is different from the probability that the trained model M2 originally targets. However, it is difficult to correctly calculate the invasive arterial pressure value, based on the invasive arterial pressure waveform data from which the pulse rate cannot be correctly calculated. Therefore, by using the trained model M2 capable of accurately estimating the probability that the pulse rate will be correctly calculated, it is also possible to accurately estimate the probability that the invasive arterial pressure value will be correctly calculated based on the invasive arterial pressure waveform data acquired from the subject being tested.

The estimation apparatus 40 can include a data processing device 44. The data processing device 44 is configured to execute an invasive arterial pressure calculation algorithm for automatically calculating the invasive arterial pressure value (at least one of a diastolic blood pressure value, a systolic blood pressure value and a mean blood pressure value) based on the invasive arterial pressure waveform data B2 received by the input interface 41.

That is, the data processing device 44 is configured to execute processing (STEP 26, in FIG. 6) of generating invasive arterial pressure value data BP1 corresponding to the heart rate automatically calculated based on the invasive arterial pressure waveform data B2 acquired from the subject being tested.

In addition, the data processing device 44 is configured to generate processed invasive arterial pressure value data BP2 by applying the processing based on the estimation data I2 output from the output interface 43 to the invasive arterial pressure value data BP1.

That is, the data processing device 44 is configured to execute processing (STEP 27, in FIG. 6) based on a probability that the invasive arterial pressure value will be correctly calculated based on the invasive arterial pressure waveform data B2, for the invasive arterial pressure value automatically calculated based on the invasive arterial pressure waveform data B2 acquired from the subject being tested.

For example, the processed invasive arterial pressure value data BP2 may be configured so that data of the invasive arterial pressure value data BP1 of which the probability that the invasive arterial pressure value is correctly calculated exceeds a predetermined threshold value, and data of which the probability does not exceed the predetermined threshold value are displayed with colors different from each other. When the aspects are different depending on whether the probability exceeds a predetermined threshold value, shapes of symbols or presence or absence of blinking indicative of the data may be appropriately selected.

Alternatively, the processed invasive arterial pressure value data BP2 may be configured so that only data of the invasive arterial pressure value data BP1 of which the probability that the invasive arterial pressure value is correctly calculated exceeds a predetermined threshold value is displayed. In contrast, the processed invasive arterial pressure value data BP2 may be configured so that only data of the invasive arterial pressure value data BP1 of which the probability that the invasive arterial pressure value is correctly calculated does not exceed a predetermined threshold value is displayed.

The estimation apparatus 40 can include a display device (not illustrated). In this case, the display corresponding to the processed invasive arterial pressure value data BP2 is presented to the display device. The display corresponding to the processed invasive arterial pressure value data BP2 may be performed in an external apparatus including a display device. In this case, the estimation apparatus 40 is configured to transmit the processed invasive arterial pressure value data BP2 to the external apparatus via a communication interface (not illustrated).

The data processing device 44 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The data processing device 44 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The data processing device 44 may also be implemented by a combination of one or more general-purpose microprocessor and a dedicated integrated circuit.

When the one or more processors 42 and the data processing device 44 are provided as devices independent of each other, the output interface 43 may be a physical communication interface configured to relay data communication therebetween. The one or more processors 42 and the data processing device 44 may also be functional entities implemented in the same control device. In this case, the output interface 43 may be a logical interface.

According to the configuration as described above, it is possible to accurately extract the invasive arterial pressure value resulting from the physiological phenomenon of the subject being tested by using a highly accurate estimation result about the probability that the invasive arterial pressure value will be correctly calculated when the invasive arterial pressure waveform data B2 acquired from the subject being tested is input to the invasive arterial pressure calculation algorithm.

In the present example, the estimation apparatus 40 is configured to estimate the probability that the invasive arterial pressure value will be correctly calculated based on the invasive arterial pressure waveform data B2 acquired from the subject being tested. In a case where the trained model M2 configured to estimate a probability that the pulse rate will be correctly calculated based on input ventricular pressure waveform data is used, the estimation apparatus 40 may be configured to estimate a probability that a ventricular pressure will be correctly calculated based on the ventricular pressure waveform data acquired from the subject being tested. The ventricular pressure is an example of the physiological parameter.

In the present example, the estimation apparatus 40 is configured to estimate the probability that the invasive arterial pressure value will be correctly calculated based on the invasive arterial pressure waveform data B2 invasively acquired from the subject being tested
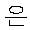 , the subject being tested. However, the estimation apparatus 40 may also be configured to estimate a probability that a predetermined physiological parameter will be correctly calculated based on noninvasive arterial pressure waveform data noninvasively acquired from the subject being tested.

For example, when the trained model M2 is generated so as to estimate a probability that the pulse rate will be correctly calculated based on the noninvasive arterial pressure waveform data, the estimation apparatus 40 may estimate a probability that a noninvasive blood pressure value (at least one of a diastolic blood pressure value and a systolic blood pressure value) will be correctly calculated based on the noninvasive arterial pressure waveform data acquired from the subject being tested, by using the trained model M2.

For example, when the trained model M2 is generated so as to estimate a probability that the pulse rate will be correctly calculated based on the pulse wave waveform data, the estimation apparatus 40 may estimate a probability that an arterial blood oxygen saturation (SpO₂) will be correctly calculated based on the pulse wave waveform data acquired from the subject being tested, by using the trained model M2.

In the present example, the estimation apparatus 40 is configured to estimate a probability that a value of a physiological parameter different from a physiological parameter relating to a probability to be estimated by the trained model M2 will be correctly calculated, by using the trained model M2 generated based on different physiological parameters acquired by different methods. However, the estimation apparatus 40 may also be configured to estimate a probability that a value of a physiological parameter different from a physiological parameter relating to a probability to be estimated by the trained model M1 will be correctly calculated, by using the trained model M1 generated based on the same type of physiological parameters acquired from the common waveform data.

For example, the estimation apparatus 40 may be configured to estimate a probability that an ST value, a QT interval and QTc will be correctly calculated based on the electrocardiogram waveform data acquired from the subject being tested, by using the trained model M1 generated based on the common electrocardiogram waveform data E1 and configured to estimate the probability that the heart rate will be correctly calculated based on the input electrocardiogram waveform data.

For example, the estimation apparatus 40 may be configured to estimate a probability that an end-tidal carbon dioxide partial pressure (EtCO2) will be correctly calculated based on the capnogram data acquired from the subject being tested, by using the trained model M1 generated based on the common capnogram data and configured to estimate a probability that a respiration rate will be correctly calculated based on the input capnogram data.

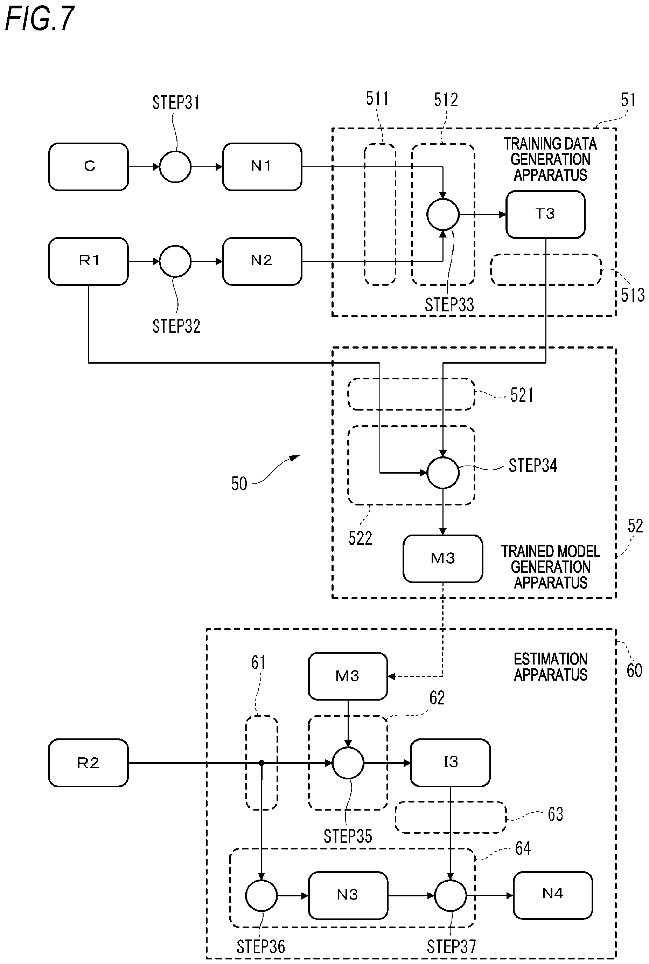

FIG. 7 exemplifies a functional configuration of a trained model generation system 50 in accordance with a third embodiment. The trained model generation system 50 is a system configured to generate a trained model M3 that is a processing algorithm, which is executed in an estimation apparatus 60 to be described later. The trained model generation system 50 can include a training data generation apparatus 51 and a trained model generation apparatus 52.

The training data generation apparatus 51 can include an input interface 511. The input interface 511 is configured to receive capnogram data C and impedance respiration waveform data R1. The input interface 511 is an example of the first input interface.

The capnogram data C is acquired from the subject being tested by using a capnometer and the like. The capnogram data C corresponds to change over time in carbon dioxide concentration in exhaled breath of the subject being tested. First respiration rate data N1 is data indicative of a value of a respiration rate correctly calculated from the capnogram data C. The respiration rate is a number of respirations made for a predetermined time by the subject being tested. The capnogram data C is an example of the first waveform data. The respiration rate is an example of the first physiological parameter. The first respiration rate data N1 is an example of the first data.

That is, when generating the trained model M3, processing (STEP 31, in FIG. 7) of acquiring the first respiration rate data N1 from the capnogram data C is performed. The counting of the respiration rate corresponding to the first respiration rate data N1 may be performed by visually checking the capnogram data C or may be performed using appropriate counting software.

The impedance respiration waveform data R1 corresponds to change over time in impedance between a plurality of electrodes attached to the subject being tested. The capnogram data C and the impedance respiration waveform data R1 are required to be acquired from the same subject being tested at the same time

Second respiration rate data N2 is data indicative of a value of the respiration rate automatically calculated from the impedance respiration waveform data R1 by the specific respiration rate calculation algorithm. The respiration rate calculation algorithm may be executed by one or more processors mounted in an electrocardiogram monitor, for example. The respiration rate calculation algorithm is configured to automatically calculate the respiration rate, based on variation timing of an impedance value of the input impedance respiration waveform data, for example. The impedance respiration waveform data R1 is an example of the second waveform data. The respiration rate is an example of the second physiological parameter. The second respiration rate data N2 is an example of the second data.

That is, when generating the trained model M3, processing (STEP 32, in FIG. 7) of acquiring the second respiration rate data N2 from the impedance respiration waveform data R1 is performed.

As exemplified in FIG. 7, the training data generation apparatus 51 can include one or more processors 512. The one or more processors 512 is configured to generate training data T3 including a training label indicating whether a value of the respiration rate corresponding to the second respiration rate data N2 is a correct answer or an incorrect answer by comparing the second respiration rate data N2 with the first respiration rate data N1. The one or more processors 512 are an example of the first one or more processors. The training data T3 is an example of the third data.

In order to include a training label indicative of a correct answer, it is not necessarily required that the respiration rate corresponding to the first respiration rate data N1 should coincide with the respiration rate corresponding to the second respiration rate data N2. When an error of the respiration rate corresponding to the second respiration rate data N2 with respect to the respiration rate corresponding to the first respiration rate data N1 is within an allowable range, the training data T3 can include training data indicative of a correct answer.

That is, when generating the trained model M3, processing (STEP 33, in FIG. 7) of comparing the second respiration rate data N2 corresponding to the respiration rate automatically calculated by the respiration rate calculation algorithm with the first respiration rate data N1 to generate the training data T3 including the training label indicating whether the respiration rate is a correct answer or an incorrect answer is performed.

The one or more processors 512 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 512 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The one or more processors 512 may also be implemented by a combination of one or more general-purpose microprocessor and a dedicated integrated circuit.

As exemplified in FIG. 7, the training data generation apparatus 51 can include an output interface 513. The output interface 513 is configured to output the training data T3 generated by the one or more processors 512.

The trained model generation apparatus 52 can include an input interface 521. The input interface 521 is configured to receive the impedance respiration data R1 and the training data T3. The input interface 521 is an example of the second input interface.

The trained model generation apparatus 52 can include one or more processors 522. The one or more processors 522 are configured to generate the trained model M3 by training the neural network with the impedance respiration waveform data R1 and the training data T3. The one or more processors 522 are an example of the second one or more processors.

The trained model M3 is generated as a processing algorithm that uses the impedance respiration waveform data as an input and outputs estimation data corresponding to a probability that the respiration rate will be correctly calculated based on the impedance respiration waveform data.

That is, when generating the learned model M3, processing (STEP 34, in FIG. 7) for training the neural network by using the impedance respiration waveform data R1 and the training data T3 is performed. As processing for training the neural network, a method relating to a well-known supervised learning is used as appropriate.

The one or more processors 522 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 522 may also be implemented by a dedicated integrated circuit such as a microcontroller, an ASIC, an FPGA, a TPU and the like capable of executing the computer program. In this case, the computer program is pre-installed in a storage device included in the dedicated integrated circuit. The storage device is an example of the storage medium in which the computer program is stored. The one or more processors 522 may also be implemented by a combination of one or more general-purpose microprocessor and a dedicated integrated circuit.

When the training data generation apparatus 51 and the trained model generation apparatus 52 are provided as apparatuses independent of each other, the output interface 513 of the training data generation apparatus 51 and the input interface 521 of the trained model generation apparatus 52 can be connected so as to enable wired communication or wireless communication. That is, the output interface 513 and the input interface 521 may be physical communication interfaces.

The training data generation apparatus 51 and the trained model generation apparatus 52 may also be functional entities implemented in the same apparatus. In this case, at least some of the functions of the one or more processors 512 of the training data generation apparatus 51 can be implemented by the one or more processors 522 of the trained model generation apparatus 52. Also, the output interface 513 and the input interface 521 may be logical interfaces.

According to the configuration as described above, the respiration rate automatically calculated by the respiration rate calculation algorithm is provided for comparison with the respiration rate known as being correctly calculated. Therefore, the training label obtained as a result of the comparison can reflect a tendency or habit of the respiration rate calculation algorithm correctly or erroneously calculating the respiration rate with respect to the input impedance respiration waveform data R1. Since the neural network is trained using the training data T3 including the training label, the generated trained model M3 can accurately estimate a probability that the respiration rate will be correctly calculated when any impedance respiration waveform data is input to the respiration rate calculation algorithm.

Also, the impedance respiration waveform data acquired from the plurality of electrodes attached to the subject being tested can be simply acquired by commonly using the electrocardiogram electrode. However, since the respiration rate is calculated based on motion of the thorax, the respiration rate is likely to be affected by body motion and noises from the electrodes. In the meantime, in order to acquire the capnogram data corresponding to the respiration rate, it is necessary to fix a mask to the mouth of the subject being tested but relatively high reliability is obtained with respect to the accuracy of the respiration rate acquired through the mask. According to the configuration as described above, since the training data T3 is generated by comparing the respiration rates calculated from the capnogram data C1 and the impedance respiration waveform data R1 acquired at the same time from the same subject being tested, it is possible to effectively train the neural network with respect to the estimation of the probability that a respiration rate will be correctly calculated based on the impedance respiration waveform data.

Therefore, estimation accuracy as to discrimination between the value of the respiration rate resulting from the physiological phenomenon of the subject being tested and the value of the respiration rate resulting from the misanalysis of the respiration rate calculation algorithm increases.

As exemplified in FIG. 7, the trained model M3 generated by the trained model generation apparatus 52 is applied to the estimation apparatus 60.

The estimation apparatus 60 can include an input interface 61. The input interface 61 is configured to receive impedance respiration waveform data R2 acquired through the plurality of electrodes attached to the subject being tested.

The estimation apparatus 60 can include one or more processors 62. The one or more processors 62 are configured to estimate a probability that the respiration rate will be correctly calculated from the impedance respiration waveform data R2. The trained model M3 is a processing algorithm that is executed for the above-described estimation by the one or more processors 62. In this case, the trained model M3 uses the impedance respiration waveform data R2, as an input, and outputs estimation data I3 corresponding to a probability that the respiration rate will be correctly calculated based on the impedance respiration waveform data R2.

That is, the estimation apparatus 60 is configured to execute processing (STEP 35, in FIG. 7) of estimating a probability that the respiration rate will be correctly calculated based on the impedance respiration waveform data R2 acquired from the subject being tested.

The one or more processors 62 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The one or more processors 62 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The one or more processors 62 may also be implemented by a combination of one or more general-purpose microprocessor and a dedicated integrated circuit.

The estimation apparatus 60 can include an output interface 63. The output interface 63 is configured to output the estimation data I3.

According to the configuration as described above, since the trained model M3, which can accurately estimate the probability that the respiration rate will be correctly calculated when the impedance respiration data is input to the respiration rate calculation algorithm, is used, estimation as to discrimination between the value of the respiration rate resulting from the physiological phenomenon of the subject being tested and the value of the respiration rate resulting from the misanalysis of the respiration rate calculation algorithm can be accurately performed by the estimation apparatus 60.

The estimation apparatus 60 can include a data processing device 64. The data processing device 64 is configured to execute the respiration rate calculation algorithm for automatically calculating the respiration rate, based on the impedance respiration waveform data R2 received by the input interface 61. In the present example, the respiration rate calculation algorithm that is executed by the data processing device 64 is the same as the respiration rate calculation algorithm used so as to acquire the second respiration rate data N2 when generating the trained model M3. Thereby, respiration rate data N3 corresponding to the respiration rate automatically calculated based on the impedance respiration waveform data R2 is generated.

That is, the data processing device 64 is configured to execute processing (STEP 36, in FIG. 7) of generating the respiration rate data N3 corresponding to the respiration rate automatically calculated based on the impedance respiration waveform data R2 acquired from the subject being tested.

In addition, the data processing device 64 is configured to generate processed respiration rate data N4 by applying the processing based on the estimation data I3 output from the output interface 63 to the respiration rate data N3.

That is, the data processing device 64 is configured to execute processing (STEP 37, in FIG. 6) based on the probability that the respiration rate will be correctly calculated based on the impedance respiration waveform data R2, for the respiration rate automatically calculated based on the impedance respiration waveform data R2 acquired from the subject being tested.

For example, the processed respiration rate data N4 may be configured so that data of the respiration rate data N3 of which the probability that the respiration rate is correctly calculated exceeds a predetermined threshold value, and data of which the probability does not exceed the predetermined threshold value are displayed with colors different from each other. When the aspects are different depending on whether the probability exceeds a predetermined threshold value, shapes of symbols or presence or absence of blinking indicative of the data may be appropriately selected.

Alternatively, the processed respiration rate data N4 may be configured so that only data of the respiration rate data N3 of which the probability that the respiration rate is correctly calculated exceeds a predetermined threshold value is displayed. In contrast, the processed respiration rate data N4 may also be configured so that only data of the respiration rate data N3 of which the probability that the respiration rate is correctly calculated does not exceed the predetermined threshold value is displayed.

The estimation apparatus 60 can include a display device (not illustrated). In this case, the display corresponding to the processed respiration rate data N4 is presented to the display device. The display corresponding to the processed respiration rate data N4 may be performed in an external apparatus including a display device. In this case, the estimation apparatus 60 is configured to transmit the processed respiration rate data N4 to the external apparatus via a communication interface (not illustrated).

The data processing device 64 having the function as described above may be implemented by one or more general-purpose microprocessors configured to operate in cooperation with one or more general-purpose memories. As the one or more general-purpose microprocessors, a CPU, an MPU and a GPU may be exemplified. As the one or more general-purpose memories, a ROM and a RAM may be exemplified. In this case, a computer program configured to execute the above-described processing may be stored in the ROM. The ROM is an example of the storage medium in which the computer program is stored. The one or more general-purpose microprocessors are configured to designate at least a part of the computer program stored on the ROM and to develop the same on the RAM, thereby executing the above-described processing in cooperation with the RAM. The computer program may be pre-installed in the one or more general-purpose memories or may be downloaded from an external server via a communication network and installed in the one or more general-purpose memories. In this case, the external server is an example of the storage medium in which the computer program is stored.

The data processing device 64 may also be implemented by a dedicated integrated circuit such as a microcontroller, an ASIC, an FPGA and the like capable of executing the computer program. In this case, the computer program is pre-installed in a storage device included in the dedicated integrated circuit. The storage device is an example of the storage medium in which the computer program is stored. The data processing device 64 may also be implemented by a combination of one or more general-purpose microprocessor and a dedicated integrated circuit.

When the one or more processors 62 and the data processing device 64 are provided as devices independent of each other, the output interface 63 may be a physical communication interface configured to relay data communication therebetween. The one or more processors 62 and the data processing device 64 may also be functional entities implemented in the same control device. In this case, the output interface 63 may be a logical interface.

According to the configuration as described above, it is possible to accurately extract the respiration rate resulting from the physiological phenomenon of the subject being tested by using a highly accurate estimation result about the probability that the respiration rate will be correctly calculated when the impedance respiration waveform data R2 acquired from the subject being tested is input to the respiration rate calculation algorithm.

The embodiments are just exemplary for easy understanding of the presently disclosed subject matter. The configurations of the embodiments can be appropriately changed and improved without departing from the gist of the presently disclosed subject matter.

The embodiments are summarized as follows.

In a first aspect of the embodiments, a method for generating a trained model is applied to an estimation apparatus configured to estimate a probability that a value of a predetermined physiological parameter is correctly calculated based on waveform data acquired from a subject being tested. The method includes: acquiring first data corresponding to a value of a first physiological parameter that has been correctly calculated from first waveform data; inputting second waveform data to an algorithm automatically calculating a value of a second physiological parameter acquired from input waveform data and to output second data; generating third data including a training label indicating whether the value of the second physiological parameter corresponding to the second data is a correct answer or an incorrect answer by comparing the second data with the first data; and training a neural network by using the second waveform data and the third data, to generate a trained model.

In a second aspect of the embodiments, a system for generating a trained model is applied to an estimation apparatus configured to estimate a probability that a value of a predetermined physiological parameter is correctly calculated based on waveform data acquired from a subject being tested. The system includes: a training data generation apparatus; and a trained model generation apparatus. The training data generation apparatus includes: a first input interface configured to receive first data corresponding to a value of a first physiological parameter that has been correctly calculated from first waveform data, and second data calculated by inputting second waveform data to an algorithm automatically calculating a value of a second physiological parameter acquired from input waveform data and to output second data; first one or more processors configured to generate third data including a training label indicating whether the value of the second physiological parameter corresponding to the second data is a correct answer or an incorrect answer by comparing the second data with the first data; and an output interface configured to output the third data. The trained model generation apparatus includes: a second input interface configured to receive the second waveform data and the third data; and second one or more processors configured to train a neural network by using the second waveform data and the third data to generate a trained model.

In a third aspect of the embodiments, a computer program is executed in a system including a training data generation apparatus and a trained model generation apparatus. When executed, the computer program causes the training data generation apparatus to: receive first data corresponding to a value of a first physiological parameter that has been correctly calculated from first waveform data, and second data acquired by inputting second waveform data to an algorithm automatically calculating a value of a second physiological parameter acquired from input waveform data and output second data; generate third data including a training label indicating whether the value of the second physiological parameter corresponding to the second data is a correct answer or an incorrect answer by comparing the second data with the first data; and output the third data. The computer program causes the trained model generation apparatus to: receive the second waveform data and the third data; and train a neural network by using the second waveform data and the third data to generate a trained model applied to an estimation apparatus configured to estimate a probability that a value of a predetermined physiological parameter is correctly calculated based on waveform data acquired from a subject being tested.

According to the configuration described above, the value of the second physiological parameter automatically calculated by the algorithm is provided for comparison with the value of the first physiological parameter known as being correctly calculated. Therefore, the training label obtained as a result of the comparison can reflect a tendency or habit of the algorithm correctly or erroneously calculating the value of the second physiological parameter with respect to the input second waveform data. Since a neural network is trained using the training data including the training label, the generated trained model can accurately estimate a probability that the value of the second physiological parameter is correctly calculated when any second waveform data is input to the algorithm. In other words, estimation accuracy as to discrimination between the value of the second physiological parameter resulting from the physiological phenomenon of the subject being tested and the value of the second physiological parameter resulting from misanalysis of the algorithm increases.

In a fourth aspect of the embodiments, an estimation apparatus includes: an input interface configured to receive waveform data acquired from a subject being tested; one or more processors configured to generate estimation data corresponding to a probability that a value of a predetermined physiological parameter is correctly calculated based on the waveform data; and an output interface configured to output the estimation data. The one or more processors are configured to generate the estimation data by using the trained model generated by the method described above.

In a fifth aspect of the embodiments, a computer program is executed by an estimation apparatus configured to estimate a probability that a value of a predetermined physiological parameter is correctly calculated based on waveform data acquired from a subject being tested. When executed, the computer program causes the estimation apparatus to: receive waveform data acquired from the subject being tested;
input the waveform data to the trained model generated by the method described above; generate estimation data corresponding to the probability, based on an output from the trained model; and output the estimation data.

According to the configuration described above, since the trained model capable of accurately estimating the probability that the predetermined physiological parameter is correctly calculated when the waveform data is input to the algorithm is used, the estimation as to discrimination between the value of the physiological parameter resulting from the physiological phenomenon of the subject being tested and the value of the physiological parameter resulting from misanalysis of the algorithm can be accurately performed by the estimation apparatus.

## Claims

1. A computer implemented method for generating a trained model (M2) to be applied to an estimation apparatus (40) for estimating a probability that a value of a predetermined physiological parameter is correctly calculated, the calculation being based on waveform data (B2) acquired from a subject being tested, the method comprising:
acquiring first data (H) corresponding to a value of a first physiological parameter that has been correctly calculated from first waveform data (E);
inputting second waveform data (B1) to an algorithm automatically calculating a value of a second physiological parameter acquired from input waveform data and to output corresponding second data (P);
generating third data (T2) including a training label indicating whether the value of the second physiological parameter corresponding to the second data (P) is a correct answer or an incorrect answer by comparing the second data (P) with the first data (H); and
training a neural network by using the second waveform data (B1) and the third data (T2), to generate the trained model (M2) which, when applied to said estimation apparatus, configures the apparatus to estimate said probability.

2. The method according to Claim 1, wherein the first waveform data (E1) also serves as the second waveform data (E1), and
wherein a type of the first physiological parameter and a type of the second physiological parameter are the same.

3. The method according to Claim 2, wherein the first waveform data (E1) is electrocardiogram waveform data, and
wherein the type of the first physiological parameter and the type of the second physiological parameter are a heart rate.

4. The method according to Claim 1, wherein the first waveform data (E) and the second waveform data (B1) are acquired from the same subject being tested by different methods, and
wherein a type of the first physiological parameter and a type of the second physiological parameter are different.

5. The method according to Claim 4, wherein the first waveform data (E) is electrocardiogram waveform data,
wherein the second waveform data (B1) is invasive arterial pressure waveform data,
wherein the type of the first physiological parameter is a heart rate, and
wherein the type of the second physiological parameter is a pulse rate.

6. The method according to Claim 1, wherein the first waveform data (C) and the second waveform data (R1) are acquired from the same subject being tested by different methods, and
wherein a type of the first physiological parameter and a type of the second physiological parameter are the same.

7. The method according to Claim 6, wherein the first waveform data (C) is capnogram waveform data,
wherein the second waveform data (R1) is impedance respiration waveform data, and
wherein the type of the first physiological parameter and the type of the second physiological parameter are a respiration rate.

8. A system for generating a trained model (M2) to be applied to an estimation apparatus (40) for estimating a probability that a value of a predetermined physiological parameter is correctly calculated, the calculation being based on waveform data (B2) acquired from a subject being tested, the system comprising:
a training data generation apparatus (31); and
a trained model generation apparatus (32),
wherein the training data generation apparatus (31) comprises:
a first input interface (311) configured to receive first data (H) corresponding to a value of a first physiological parameter that has been correctly calculated from first waveform data (E), and second data (P) calculated by inputting second waveform data (B1) to an algorithm automatically calculating a value of a second physiological parameter acquired from input waveform data (B1) and to output corresponding second data (P);
first one or more processors (312) configured to generate third data (T2) including a training label indicating whether the value of the second physiological parameter corresponding to the second data (P) is a correct answer or an incorrect answer by comparing the second data (P) with the first data (H); and
an output interface (313) configured to output the third data (T2), and
wherein the trained model generation apparatus (32) comprises:
a second input interface (321) configured to receive the second waveform data (B1) and the third data (T2); and
second one or more processors (322) confiaured to train a neural network bv using the second waveform data (B1) and the third data (T2) to generate the trained model (M2) which, when applied to said estimation apparatus, configures the apparatus to estimate said probability.

9. A computer program for execution in a system comprising a training data generation apparatus (31) and a trained model generation apparatus (32), wherein
when executed,
the computer program causes the training data generation apparatus (31) to:
receive first data (H) corresponding to a value of a first physiological parameter that has been correctly calculated from first waveform data (E), and second data (P) acquired by inputting second waveform data (B1) to an algorithm automatically calculating a value of a second physiological parameter acquired from input waveform data (B1) and output corresponding second data (P);
generate third data (T2) including a training label indicating whether the value of the second physiological parameter corresponding to the second data (P) is a correct answer or an incorrect answer by comparing the second data (P) with the first data (H); and
output the third data (T2), and
the computer program causes the trained model generation apparatus (32) to:
receive the second waveform data (B1) and the third data (T2), and
train a neural network by using the second waveform data (B1) and the third data (T2) to generate a trained model (M2) which, when applied to an estimation apparatus (40) for estimating a probability that a value of a predetermined physiological parameter is correctly calculated, configures the estimation apparatus to estimate a probability that a value of a predetermined physiological parameter is correctly calculated, the calculation being based on waveform data (B2) acquired from a subject being tested.

10. An estimation apparatus (40) comprising:
an input interface (41) configured to receive waveform data (B2) acquired from a subject being tested;
one or more processors (42) configured to generate estimation data (I2) corresponding to a probability that a value of a predetermined physiological parameter is correctly calculated, the calculation being based on the waveform data (B2); and
an output interface (43) configured to output the estimation data (I2),
wherein the one or more processors (42) are configured to generate the estimation data (I2) by using the trained model (M2) generated by the method according to any one of Claims 1 to 7.

11. The estimation apparatus (40) according to Claim 10, further comprising a data processing device (44) configured to execute an algorithm outputting, as output data, the value of the predetermined physiological parameter automatically calculated based on the waveform data,
wherein the data processing device (44) is configured to apply processing based on the estimation data (12) to the output data.

12. The estimation apparatus (40) according to Claim 10 or 11, wherein a type of the predetermined physiological parameter is the same as a type of the physiological parameter used for generating of the trained model (M2).

13. The estimation apparatus (40) according to Claim 10 or 11, wherein a type of the predetermined physiological parameter is different from a type of the physiological parameter used for generating of the trained model (M2).

14. A computer program executed by an estimation apparatus (40) configured to estimate a probability that a value of a predetermined physiological parameter is correctly calculated, the calculation being based on waveform data (B2) acquired from a subject being tested, wherein
when executed, the computer program causes the estimation apparatus (40) to:
receive waveform data (B2) acquired from the subject being tested;
input the waveform data (B2) to a trained model (M2) that was generated by the method according to any one of Claims 1 to 7;
generate estimation data (I2) corresponding to the probability, based on an output from the trained model (M2); and
output the estimation data (I2).

## Patentansprüche

1. Computerimplementiertes Verfahren zum Erzeugen eines trainierten Modells (M2), das bei einer Schätzungsvorrichtung (40) eingesetzt werden soll, mit der eine Wahrscheinlichkeit geschätzt wird, dass ein Wert eines vorgegebenen physiologischen Parameters richtig berechnet wird, wobei die Berechnung auf Wellenform-Daten (B2) basiert, die von einem getesteten Patienten ermittelt werden, und das Verfahren umfasst:
Bestimmen erster Daten (H), die einem Wert eines ersten physiologischen Parameters entsprechen, der anhand erster Wellenform-Daten (E) richtig berechnet worden ist;
Eingeben zweiter Wellenform-Daten (B1) in einen Algorithmus, der automatisch einen anhand eingegebener Wellenform-Daten ermittelten Wert eines zweiten physiologischen Parameters berechnet und entsprechende zweite Daten (P) ausgibt;
Erzeugen dritter Daten (T2), die einen Kennsatz enthalten, der anzeigt, ob der Wert des zweiten physiologischen Parameters, der den zweiten Daten (P) entspricht, eine richtige Antwort oder eine unrichtige Antwort ist, durch Vergleichen der zweiten Daten (P) mit den ersten Daten (H); sowie
Trainieren eines neuronalen Netzes unter Verwendung der zweiten Wellenform-Daten (B1) und der dritten Daten (T2), um das trainierte Modell (M2) zu erzeugen, das, wenn es bei der Schätzungsvorrichtung eingesetzt wird, die Vorrichtung zum Schätzen der Wahrscheinlichkeit konfiguriert.

2. Verfahren nach Anspruch 1, wobei die ersten Wellenform-Daten (E1) auch als die zweiten Wellenform-Daten (E1) dienen, und
ein Typ des ersten physiologischen Parameters und ein Typ des zweiten physiologischen Parameters gleich sind.

3. Verfahren nach Anspruch 2, wobei die ersten Wellenform-Daten (E1) Wellenform-Daten eines Elektrokardiogramms sind, und
der Typ des ersten physiologischen Parameters und der Typ des zweiten physiologischen Parameters eine Herzfrequenz sind.

4. Verfahren nach Anspruch 1, wobei die ersten Wellenform-Daten (E) und die zweiten Wellenform- Daten (B1) von dem gleichen Patienten ermittelt werden, der mit unterschiedlichen Verfahren getestet wird, und
wobei ein Typ des ersten physiologischen Parameters und ein Typ des zweiten physiologischen Parameters verschieden sind.

5. Verfahren nach Anspruch 4, wobei die ersten Wellenform-Daten (E) Wellenform-Daten eines Elektrokardiogramms sind,
die zweiten Wellenform-Daten (B1) Wellenform-Daten von invasiv ermitteltem arteriellem Blutdruck sind,
der Typ des ersten physiologischen Parameters eine Herzfrequenz ist, und
der Typ des zweiten physiologischen Parameters eine Pulsfrequenz ist.

6. Verfahren nach Anspruch 1, wobei die ersten Wellenform-Daten (C) und die zweiten Wellenform- Daten (R1) von dem gleichen Patienten ermittelt werden, der mit unterschiedlichen Verfahren getestet wird, und
ein Typ des ersten physiologischen Parameters und ein Typ des zweiten physiologischen Parameters gleich sind.

7. Verfahren nach Anspruch 6, wobei die ersten Wellenform-Daten (C) Wellenform-Daten eines Kapnogramms sind,
die zweiten Wellenform-Daten (R1) Wellenform-Daten des Atemwiderstandes sind, und
der Typ des ersten physiologischen Parameters und der Typ des zweiten physiologischen Parameters eine Atemfrequenz sind.

8. System zum Erzeugen eines trainierten Modells (M2), das bei einer Schätzungsvorrichtung (40) eingesetzt werden soll, mit der eine Wahrscheinlichkeit geschätzt wird, dass ein Wert eines vorgegebenen physiologischen Parameters richtig berechnet wird, wobei die Berechnung auf Wellenform-Daten (B2) basiert, die von einem getesteten Patienten ermittelt werden, und das System umfasst:
eine Vorrichtung (31) für Erzeugung von Trainingsdaten; sowie
eine Vorrichtung (32) für Erzeugung eines trainierten Modells,
wobei die Vorrichtung (31) für Erzeugung von Trainingsdaten umfasst:
eine erste Eingabe-Schnittstelle (311), die so konfiguriert ist, dass sie erste Daten (H), die einem Wert eines ersten physiologischen Parameters entsprechen, der anhand erster Wellenform-Daten (E) richtig berechnet worden ist, sowie zweite Daten (P) empfängt, die durch Eingeben zweiter Wellenform-Daten (B1) in einen Algorithmus berechnet werden, der automatisch einen Wert eines zweiten physiologischen Parameters berechnet, der anhand eingegebener Wellenform-Daten (B1) bestimmt worden ist, und entsprechende zweite Daten (P) ausgibt;
einen oder mehrere ersten/erste Prozessor/en, der/die so konfiguriert ist/sind, dass er/sie durch Vergleichen der zweiten Daten (P) mit den ersten Daten (H) dritte Daten (T2) erzeugt/erzeugen, die einen Kennsatz enthalten, der anzeigt, ob der Wert des zweiten physiologischen Parameters, der den zweiten Daten (P) entspricht, eine richtige Antwort oder eine unrichtige Antwort ist, sowie
eine Ausgabe-Schnittstelle (313), die zum Ausgeben der dritten Daten (T2) konfiguriert ist, und
wobei die Vorrichtung (32) für Erzeugung eines trainierten Modells umfasst:
eine zweite Eingabe-Schnittstelle (321), die zum Empfangen der zweiten Wellenform-Daten (B1) und der dritten Daten (T2) konfiguriert ist; sowie
einen oder mehrere zweiten/zweite Prozessor/en, der/die so konfiguriert ist/sind, dass er/sie ein neuronales Netz unter Verwendung der zweiten Wellenform-Daten (B1) und der dritten Daten (T2) trainiert/trainieren, um das trainierte Modell (M2) zu erzeugen, das, wenn es auf die Schätzungsvorrichtung angewendet wird, die Vorrichtung zum Schätzen der Wahrscheinlichkeit konfiguriert.

9. Computerprogramm zur Ausführung in einem System, das eine Vorrichtung (31) für Erzeugung von Trainingsdaten sowie eine Vorrichtung (32) für Erzeugung eines trainierten Modells umfasst,
wobei das Programm, wenn es ausgeführt wird, die Vorrichtung (31) für Erzeugung von Trainingsdaten veranlasst durchzuführen:
Empfangen erster Daten (H), die einem Wert eines ersten physiologischen Parameters entsprechen, der anhand erster Wellenform-Daten (E) richtig berechnet worden ist, sowie zweiter Daten (P), die durch Eingeben zweiter Wellenform-Daten (B1) in einen Algorithmus ermittelt werden, der automatisch einen anhand eingegebener Wellenform-Daten (B1) ermittelten Wert eines zweiten physiologischen Parameters berechnet und entsprechende zweite Daten (P) ausgibt;
Erzeugen dritter Daten (T2), die einen Kennsatz enthalten, der anzeigt, ob der Wert des zweiten physiologischen Parameters, der den zweiten Daten (P) entspricht, eine richtige Antwort oder eine unrichtige Antwort ist, durch Vergleichen der zweiten Daten (P) mit den ersten Daten (H); sowie
Ausgeben der dritten Daten (T2), und
wobei das Computerprogramm die Vorrichtung (32) für Erzeugung eines trainierten Modells veranlasst durchzuführen:
Empfangen der zweiten Wellenform-Daten (B1) und der dritten Daten (T2), sowie
Trainieren eines neuronalen Netzes unter Verwendung der zweiten Wellenform-Daten (B1) und der dritten Daten (T2), um ein trainiertes Modell (M2) zu erzeugen, das, wenn es bei einer Schätzungsvorrichtung (40) eingesetzt wird, mit der eine Wahrscheinlichkeit geschätzt wird, dass ein Wert eines vorgegebenen physiologischen Parameters richtig berechnet wird, die Schätzungsvorrichtung so konfiguriert, dass sie eine Wahrscheinlichkeit schätzt, dass ein Wert eines vorgegebenen physiologischen Parameters richtig berechnet wird, wobei die Berechnung auf Wellenform-Daten (B2) basiert, die von einem getesteten Patienten ermittelt werden.

10. Schätzungsvorrichtung (40), die umfasst:
eine Eingabe-Schnittstelle (41), die zum Empfangen von einem getesteten Patienten ermittelter Wellenform-Daten (B2) konfiguriert ist;
einen oder mehrere Prozessor/en (42), der/die so konfiguriert ist/sind, dass er/sie Schätzungs-Daten (12) erzeugt/erzeugen, die einer Wahrscheinlichkeit entsprechen, dass ein Wert eines vorgegebenen physiologischen Parameters richtig berechnet wird, wobei die Berechnung auf den Wellenform-Daten (B2) basiert; sowie
eine Ausgabe-Schnittstelle (43), die zum Ausgeben der Schätzungs-Daten (12) konfiguriert ist, und
wobei der eine oder die mehreren Prozessor/en (42) so konfiguriert ist/sind, dass er/sie die Schätzungs-Daten (12) unter Verwendung des mit dem Verfahren nach einem der Ansprüche 1 bis 7 erzeugten trainierten Modells (M2) erzeugt/erzeugen.

11. Schätzungsvorrichtung (40) nach Anspruch 10, die des Weiteren eine Datenverarbeitungseinrichtung (44) umfasst, die zum Ausführen eines Algorithmus konfiguriert ist, der als Ausgabe-Daten den Wert des vorgegebenen physiologischen Parameters ausgibt, der auf Basis der Wellenform-Daten automatisch berechnet wird,
wobei die Datenverarbeitungseinrichtung (44) zum Durchführen auf den Schätzungs-Daten (12) basierender Verarbeitung an den Ausgabe-Daten konfiguriert ist.

12. Schätzungsvorrichtung (40) nach Anspruch 10 oder 11, wobei ein Typ des vorgegebenen physiologischen Parameters der gleiche ist wie ein Typ des zum Erzeugen des trainierten Modells (M2) verwendeten physiologischen Parameters.

13. Schätzungsvorrichtung (40) nach Anspruch 10 oder 11, wobei ein Typ des vorgegebenen physiologischen Parameters sich von einem Typ des zum Erzeugen des trainierten Modells (M2) verwendeten physiologischen Parameters unterscheidet.

14. Computerprogramm, das von einer Schätzungsvorrichtung (40) ausgeführt wird, die so konfiguriert ist, dass sie eine Wahrscheinlichkeit schätzt, dass ein Wert eines vorgegebenen physiologischen Parameters richtig berechnet wird, wobei die Berechnung auf Wellenform-Daten (B2) basiert, die von einem getesteten Patienten ermittelt werden, und
wobei das Computerprogramm, wenn es ausgeführt wird, die Schätzungsvorrichtung (40) veranlasst, durchzuführen:
Empfangen von einem getesteten Patienten ermittelter Wellenform-Daten (B2);
Eingeben der Wellenform-Daten (B2) in mit dem Verfahren nach einem der Ansprüche 1 bis 7 erzeugtes trainiertes Modell (M2);
Erzeugen der Wahrscheinlichkeit entsprechender Schätzungs-Daten (12) basierend auf einem Ausgang von dem trainierten Modell (M2); und
Ausgeben der Schätzungs-Daten (12).

## Revendications

1. Procédé mis en œuvre par ordinateur pour générer un modèle entraîné (M2) à appliquer à un appareil d'estimation (40) afin d'estimer une probabilité qu'une valeur d'un paramètre physiologique prédéterminé est correctement calculée, le calcul étant basé sur des données de forme d'onde (B2) acquises auprès d'un sujet qui est testé, le procédé comprenant :
l'acquisition de premières données (H) correspondant à une valeur d'un premier paramètre physiologique qui a été correctement calculée à partir des premières données de forme d'onde (E) ;
la saisie de secondes données de forme d'onde (B1) au niveau d'un algorithme calculant automatiquement une valeur d'un second paramètre physiologique acquis à partir de données de forme d'onde d'entrée et pour délivrer de secondes données (P) correspondantes ;
la génération de troisièmes données (T2) incluant une étiquette d'apprentissage indiquant si la valeur du second paramètre physiologique correspondant aux secondes données (P) est une réponse correcte ou une réponse incorrecte en comparant les secondes données (P) aux premières données (H) ; et
l'entraînement d'un réseau neuronal en utilisant les secondes données de forme d'onde (B1) et les troisièmes données (T2), pour générer le modèle entraîné (M2) qui, lorsqu'appliqué audit appareil d'estimation, configure l'appareil pour estimer ladite probabilité.

2. Procédé selon la revendication 1, dans lequel les premières données de forme d'onde (E1) servent également de secondes données de forme d'onde (E1), et
dans lequel un type du premier paramètre physiologique et un type du second paramètre physiologique sont identiques.

3. Procédé selon la revendication 2, dans lequel les premières données de forme d'onde (E1) sont des données de forme d'onde d'électrocardiogramme, et
dans lequel le type du premier paramètre physiologique et le type du second paramètre physiologique sont une fréquence cardiaque.

4. Procédé selon la revendication 1, dans lequel les premières données de forme d'onde (E) et les secondes données de forme d'onde (B1) sont acquises auprès du même sujet qui est testé par différents procédés, et
dans lequel un type du premier paramètre physiologique et un type du second paramètre physiologique sont différents.

5. Procédé selon la revendication 4, dans lequel les premières données de forme d'onde (E) sont des données de forme d'onde d'électrocardiogramme,
dans lequel les secondes données de forme d'onde (B1) sont des données de forme d'onde de tension artérielle invasive,
dans lequel le type du premier paramètre physiologique est une fréquence cardiaque, et
dans lequel le type du second paramètre physiologique est une fréquence du pouls.

6. Procédé selon la revendication 1, dans lequel les premières données de forme d'onde (C) et les secondes données de forme d'onde (R1) sont acquises auprès du même sujet qui est testé par différents procédés, et
dans lequel un type du premier paramètre physiologique et un type du second paramètre physiologique sont identiques.

7. Procédé selon la revendication 6, dans lequel les premières données de forme d'onde (C) sont des données de forme d'onde type capnogramme,
dans lequel les secondes données de forme d'onde (R1) sont des données d'impédance de forme d'onde de respiration, et
dans lequel le type du premier paramètre physiologique et le type du second paramètre physiologique sont une fréquence respiratoire.

8. Système de génération d'un modèle entraîné (M2) à appliquer à un appareil d'estimation (40) afin d'estimer une probabilité qu'une valeur d'un paramètre physiologique prédéterminé est correctement calculée, le calcul étant basé sur des données de forme d'onde (B2) acquises auprès d'un sujet qui est testé, le système comprenant :
un appareil de génération de données d'apprentissage (31) ; et
un appareil de génération de modèle entraîné (32),
dans lequel l'appareil de génération de données d'apprentissage (31) comprend :
une première interface d'entrée (311) configurée pour recevoir de premières données (H) correspondant à une valeur d'un premier paramètre physiologique qui a été correctement calculée à partir des premières données de forme d'onde (E), et de secondes données (P) calculées par saisie de secondes données de forme d'onde (B1) au niveau d'un algorithme calculant automatiquement une valeur d'un second paramètre physiologique acquise à partir des données de forme d'onde d'entrée (B1) et pour délivrer de secondes données (P) correspondantes ;
de premiers un ou plusieurs processeurs (312) configurés pour générer de troisièmes données (T2) incluant une étiquette d'apprentissage indiquant si la valeur du second paramètre physiologique correspondant aux secondes données (P) est une réponse correcte ou une réponse incorrecte en comparant les secondes données (P) aux premières données (H) ; et
une interface de sortie (313) configurée pour délivrer les troisièmes données (T2), et
dans lequel l'appareil de génération de modèle entraîné (32) comprend :
une seconde interface d'entrée (321) configurée pour recevoir les secondes données de forme d'onde (B1) et les troisièmes données (T2) ; et
de seconds un ou plusieurs processeurs (322) configurés pour entraîner un réseau neuronal en utilisant les secondes données de forme d'onde (B1) et de troisièmes données (T2) pour générer le modèle entraîné (M2) qui, lorsqu'il est appliqué audit appareil d'estimation, configure l'appareil afin d'estimer ladite probabilité.

9. Programme informatique d'exécution dans un système comprenant un appareil de génération de données d'apprentissage (31) et un appareil de génération de modèle entraîné (32), dans lequel
lorsqu'il est exécuté,
le programme informatique entraîne l'appareil de génération de données d'apprentissage (31) à :
recevoir de premières données (H) correspondant à une valeur d'un premier paramètre physiologique qui a été correctement calculée à partir de premières données de forme d'onde (E), et de secondes données (P) acquises en saisissant de secondes données de forme d'onde (B1) au niveau d'un algorithme calculant automatiquement une valeur d'un second paramètre physiologique acquise à partir des données de forme d'onde d'entrée (B1) et de secondes données (P) correspondantes de sortie ;
générer de troisièmes données (T2) incluant une étiquette d'apprentissage indiquant si la valeur du second paramètre physiologique correspondant aux secondes données (P) est une réponse correcte ou une réponse incorrecte en comparant les secondes données (P) aux premières données (H) ; et
délivrer les troisièmes données (T2), et
le programme informatique amène l'appareil de génération de modèle entraîné (32) à :
recevoir les secondes données de forme d'onde (B1) et les troisièmes données (T2), et
à entraîner un réseau neuronal en utilisant les secondes données de forme d'onde (B1) et les troisièmes données (T2) afin de générer un modèle entraîné (M2) qui, lorsqu'il est appliqué à un appareil d'estimation (40) pour estimer une probabilité qu'une valeur d'un paramètre physiologique prédéterminé est correctement calculée, configure l'appareil d'estimation pour estimer une probabilité qu'une valeur d'un paramètre physiologique prédéterminé est correctement calculée, le calcul étant basé sur des données de forme d'onde (B2) acquises auprès d'un sujet qui est testé.

10. Appareil d'estimation (40) comprenant :
une interface d'entrée (41) configurée pour recevoir des données de forme d'onde (B2) acquises auprès d'un sujet qui est testé ;
un ou plusieurs processeurs (42) configurés pour générer des données d'estimation (12) correspondant à une probabilité qu'une valeur d'un paramètre physiologique prédéterminé est correctement calculée, le calcul étant basé sur les données de forme d'onde (B2) ; et
une interface de sortie (43) configurée pour délivrer les données d'estimation (12),
dans lequel le un ou plusieurs processeurs (42) sont configurés pour générer les données d'estimation (12) en utilisant le modèle entraîné (M2) généré par le procédé selon l'une quelconque des revendications 1 à 7.

11. Appareil d'estimation (40) selon la revendication 10, comprenant en outre un dispositif de traitement de données (44) configuré pour exécuter un algorithme délivrant, comme données délivrées, la valeur du paramètre physiologique prédéterminé calculée automatiquement sur la base des données de forme d'onde,
dans lequel le dispositif de traitement de données (44) est configuré pour appliquer un traitement sur la base des données d'estimation (12) aux données délivrées.

12. Appareil d'estimation (40) selon la revendication 10 ou 11, dans lequel un type du paramètre physiologique prédéterminé est le même qu'un type du paramètre physiologique utilisé pour générer le modèle entraîné (M2).

13. Appareil d'estimation (40) selon la revendication 10 ou 11, dans lequel un type du paramètre physiologique prédéterminé est différent d'un type du paramètre physiologique utilisé pour générer le modèle entraîné (M2).

14. Programme informatique exécuté par un appareil d'estimation (40) configuré pour estimer une probabilité qu'une valeur d'un paramètre physiologique prédéterminé est correctement calculée, le calcul étant basé sur des données de forme d'onde (B2) acquises auprès d'un sujet qui est testé, dans lequel
lorsqu'il est exécuté, le programme informatique entraîne l'appareil d'estimation (40) à :
recevoir des données de forme d'onde (B2) acquises auprès du sujet qui est testé ;
saisir les données de forme d'onde (B2) au niveau d'un modèle entraîné (M2) qui était généré par le procédé selon l'une quelconque des revendications 1 à 7 ;
générer des données d'estimation (12) correspondant à la probabilité, sur la base d'un résultat du modèle entraîné (M2) ; et
à délivrer les données d'estimation (12).
